# EUROPÄISCHE PATENTANMELDUNG

(11) **EP 1 576 971 A1**
(43) Veröffentlichungstag der Anmeldung: **21.09.2005**
(21) Anmeldenummer: 05002050.2
(22) Anmeldetag: 01.02.2005
(51) Int. Cl.: A61M 1/00, F04B 49/06, A61M 5/142

(54) **Medizinische Einmalverwendungsartikel mit Einmalverwendungssicherung**

(30) Priorität: 17.03.2004 DE 102004013159
(71) Anmelder: Möller Medical GmbH & Co.KG, 36043 Fulda (DE)
(72) Erfinder: Schröter, Werner, 36137 Grossenlüder (DE)
(74) Vertreter: Graf von Stosch, Andreas

(57) **Zusammenfassung**

Die Aufgabe medizinische Einmalverwendungsartikel zu schaffen, bei denen die Möglichkeit einer mehrfachen Verwendung ausgeschlossen, wird bei der erfindungsgemäßen Vorrichtung dadurch gelöst, dass ein medizinisches Gerät in Verbindung mit mindestens einem Einmalverwendungsartikel elektrisch betreibbar ist, wobei das medizinische Gerät mit einer Erkennungselektronik ausgestattet ist, die einen Sender und einen Empfänger umfasst, und der mindestens eine Einmalverwendungsartikel eine Einmalverwendungssicherung mit elektronischen Mitteln aufweist, die ebenfalls einen Sender und einen Empfänger zum Austausch von Daten zwischen der Einmalverwendungssicherung und der Erkennungselektronik im medizinischen Gerät umfassen, die vorzugsweise drahtlos über eine Funkverbindung erfolgt, und die Erkennungselektronik in Abhängigkeit von einer Auswertung der zwischen der Einmalverwendungssicherung der Erkennungselektronik ausgetauschten Daten den Betrieb des medizinischen Geräts steuert. Dadurch kann ein gesundheitliches Risiko für den Patienten, das durch mehrfache Verwendung von Einmalverwendungsartikel entstehen kann, minimiert werden.

## Beschreibung

Die vorliegende Erfindung bezieht sich auf medizinische Geräte, die in Verbindung mit mindestens einem Einmalverwendungsartikel betrieben werden sowie auf ein Verfahren zum Betreiben solcher medizinischen Geräte. Die Erfindung bezieht sich ferner auf medizinische Einmalverwendungsartikel als Zubehör zu aktiven, d.h. elektrisch betriebenen Geräten, die im medizinischen Bereich zur Anwendung kommen.

Zu den aktiven, elektrisch betriebenen medizinischen Geräten gehören beispielsweise Infusionspumpen, Infiltrationspumpen, Tumeszenzpumpen, Dialysegeräte und Herz-Lungen-Maschinen. Häufig werden Schlauchpumpen als Infusionspumpen oder Infiltrationspumpen verwendet, bei denen durch Einwirkung eines Rotationskolbens von außen auf einen flexiblen Schlauch eine weitgehend kontinuierliche Pumpleistung erzeugt wird, ohne dass die zu pumpende Flüssigkeit mit dem Rotationskolben in direkten Kontakt kommt.

Da im medizinischen Bereich allgemein und insbesondere bei Infusionen die Sterilität von größter Bedeutung ist, wird die zu pumpende Flüssigkeit in der Regel von der Schlauchpumpe über sterile Verbindungsschläuche zum Patienten geführt. Um die Sterilität zu gewährleisten, sollen die Verbindungsschläuche bei jedem neuen Einsatz ausgetauscht werden und bei jeder Anwendung neue Verbindungsschläuche verwendet werden. Um einen schnellen und einfachen Austausch der Verbindungsschläuche zu ermöglichen, sind die Verbindungsschläuche an der Schlauchpumpe beispielsweise über schnell lösbare Steckverbindungen anschließbar. Solche Verbindungsschläuche für Infusions-Schlauchpumpen sind sogenannte Einmalverwendungsartikel, die aus Gründen der Sterilität nur einmal verwendet werden sollen.

Bekannte Einmalverwendungsartikel haben den Nachteil, dass die sie beispielsweise versehentlich ein zweites Mal oder sogar mehrere Male verwendet werden können. Die Wiederverwendung solcher Einmalverwendungsartikel birgt jedoch ein erhebliches Gesundheitsrisiko für den Patienten.

Aufgabe der vorliegenden Erfindung ist es daher, medizinische Einmalverwendungsartikel zu schaffen, bei denen die Möglichkeit einer mehrfachen Verwendung ausgeschlossen ist, um damit die gesundheitliche Sicherheit des Patienten zu verbessern.

Diese Aufgabe wird nach der vorliegenden Erfindung durch ein medizinisches Gerät mit den Merkmalen gemäß Anspruch 1 gelöst. Die Aufgabe wird auch durch einen erfindungsgemäßen Einmalverwendungsartikel mit den in Anspruch 4 genannten Merkmalen gelöst. Diese Aufgabe wird ferner nach der vorliegenden Erfindung durch ein Verfahren mit den Merkmalen gemäß Anspruch 16 gelöst. Vorteilhafte Weiterbildungen der vorliegenden Erfindung sind in den Unteransprüchen 2, 3, 5 bis 15 und 17 bis 25 gekennzeichnet.

Ein medizinisches Gerät gemäß der vorliegenden Erfindung ist in Verbindung mit mindestens einem Einmalverwendungsartikel elektrisch betreibbar, wobei das medizinische Gerät mit einer Erkennungselektronik ausgestattet ist, die einen Sender und einen Empfänger umfasst, und der mindestens eine Einmalverwendungsartikel eine Einmalverwendungssicherung mit elektronischen Mitteln aufweist, die ebenfalls einen Sender und einen Empfänger zum Austausch von Daten zwischen der Einmalverwendungssicherung und der Erkennungselektronik im medizinischen Gerät umfassen, und die Erkennungselektronik in Abhängigkeit von einer Auswertung der zwischen der Einmalverwendungssicherung der Erkennungselektronik ausgetauschten Daten den Betrieb des medizinischen Geräts steuert. Der Austausch von Daten zwischen der Einmalverwendungssicherung und der Erkennungselektronik erfolgt dabei vorzugsweise drahtlos über eine Funkverbindung mittels elektromagnetischer Wellen.

Um ein gesundheitliches Risiko für den Patienten zu minimieren, wird der Einmalverwendungsartikel mit einer unverlierbaren Einmalverwendungssicherung in Form eines Erkennungssensors ausgestattet. Dieser Erkennungssensor ist in der Lage, über drahtlose Kommunikation mit der Erkennungselektronik des medizinischen Geräts Daten auszutauschen. Die Erkennungselektronik des medizinischen Geräts führt eine Überprüfung der aus der Einmalverwendungssicherung erhaltenen Daten durch und kann aufgrund des Ergebnis der Datenüberüberprüfung den Betrieb des medizinischen Geräts steuern. Wenn die Überprüfung der aus der Einmalverwendungssicherung erhaltenen Daten zu dem Ergebnis führt, dass der betreffende Einmalverwendungsartikel aus irgendwelchen Gründen nicht zum Einsatz geeignet oder zugelassen ist, so lässt die Erkennungselektronik den Betrieb des medizinischen Geräts nicht zu. Zu diesem Zweck ist bei einer bevorzugten Ausführungsform der vorliegenden Erfindung die Erkennungselektronik des medizinischen Geräts mit einer elektronischen Steuerung des medizinischen Geräts verbunden, um den Betrieb des medizinischen Geräts zu steuern.

In einer besonders geeigneten Anwendung der vorliegenden Erfindung ist das medizinische Gerät eine elektrisch betriebene Schlauchpumpe zur Beförderung von Fluiden und der Einmalverwendungsartikel ein Verbindungsschlauch, der von einem das zu befördernde Fluid enthaltenden Reservoir zu der Schlauchpumpe führt, sowie ein Verbindungsschlauch, der von der Schlauchpumpe zu einer Infusionsvorrichtung führt. Solche Anlagen finden beispielsweise bei Infusionspumpen, Infiltrationspumpen und Tumeszenzpumpen Anwendung, wobei dem Patienten eine Flüssigkeit von einem Reservoir über die Schlauchpumpe und eine Infusionsvorrichtung injiziert wird. Dabei führt ein Verbindungsschlauch von dem die zu befördernde Flüssigkeit enthaltenden Reservoir zu der Schlauchpumpe und ein Verbindungsschlauch von der Schlauchpumpe zu der Infusionsvorrichtung.

Die oben genannte Aufgabe wird nach der vorliegenden Erfindung auch durch einen Einmalverwendungsartikel gelöst, der in Verbindung mit einem medizinischen Gerät nach einem der vorangehenden Ansprüche einsetzbar ist, wobei der Einmalverwendungsartikel eine Einmalverwendungssicherung mit elektronischen Mitteln aufweist, die einen Sender und einen Empfänger zum Austausch von Daten zwischen der Einmalverwendungssicherung und einer Erkennungselektronik im medizinischen Gerät umfassen. Besonders vorteilhaft ist es, wenn die Einmalverwendungssicherung des Einmalverwendungsartikels einen elektronischen Erkennungssensor, beispielsweise einen Transponder umfasst, der mit dem Einmalverwendungsartikel vorzugsweise unverlierbar verbunden ist. Durch die unverlierbare Anordnung der Einmalverwendungssicherung am Einmalverwendungsartikel wird verhindert, dass die Einmalverwendungssicherung vom Einmalverwendungsartikel getrennt wird und der Einmalverwendungsartikel mehr als einmal verwendet wird.

Bei einer bevorzugten Ausführungsform der vorliegenden Erfindung umfasst der Erkennungssensor der Einmalverwendungssicherung elektronische Speichermittel, deren Dateninhalt durch die Erkennungselektronik während des Betriebs des medizinischen Geräts verändert werden kann. Aufgrund der beschreibbaren Eigenschaft des Erkennungssensors kann der Dateninhalt der elektronischen Speichermittel im Erkennungssensor durch die Erkennungselektronik des medizinischen Geräts beschrieben werden. Auf diese Weise kann die Erkennungselektronik nach oder während des Betriebs des medizinischen Geräts den Dateninhalt der elektronischen Speichermittel im Erkennungssensor dahingehend verändern, dass der Einmalverwendungsartikel benutzt wurde und dadurch den Einmalverwendungsartikel für einen weiteren Gebrauch als unbrauchbar kennzeichnen.

Zwischen der Einmalverwendungssicherung bzw. dem Erkennungssensor des Einmalverwendungsartikels und der Erkennungselektronik des medizinischen Geräts findet somit ein Datenaustausch statt. Bei einer weiteren bevorzugten Ausführungsform der vorliegenden Erfindung überprüft die Erkennungselektronik während des Datenaustauschs zwischen dem Erkennungssensor und der Erkennungselektronik des medizinischen Geräts, ob der Dateninhalt des Erkennungssensors bereits bei einer vorangegangenen Verwendung des Einmalverwendungsartikels verändert wurde bzw. ob der Dateninhalt des Erkennungssensors Hinweise auf eine vorangegangene Verwendung des Einmalverwendungsartikels enthält. Durch den Einsatz von Einmalverwendungsartikeln mit Einmalverwendungssicherung in Form eines beschreibbaren Erkennungssensors kann sichergestellt werden, dass ein Einmalverwendungsartikel tatsächlich nur einmal verwendet wird, was eine erhöhte Sicherheit für den Patienten mit sich bringt.

Zweckmäßigerweise umfasst die Einmalverwendungssicherung dazu elektronische Speichermittel mit mindestens einem vorzugsweise wiederbeschreibbaren elektronischen Register, in dem Daten gespeichert werden können. Die Veränderung des Dateninhalts des Erkennungssensors erfolgt dabei durch das beschreiben eines oder mehrerer elektronischer Register im Erkennungssensor. Dabei werden in den elektronischen Speichermitteln der Einmalverwendungssicherung des Einmalverwendungsartikels insbesondere Informationen gespeichert, die sich auf eine vorangegangene Benutzung und/oder auf eine Zeitspanne der Haltbarkeit und/oder auf die Umgebungsbedingungen während der Lagerung und/oder auf die Eignung des betreffenden Einmalverwendungsartikels für das medizinische Gerät und/oder auf den zu behandelnden Patienten beziehen. Ferner kann der Erkennungssensor der Einmalverwendungssicherung zumindest einen elektronischen Speicher umfassen, der nur einmal beschreibbar ist. Dabei kann die Erkennungselektronik den Betrieb des medizinischen Geräts verhindern, wenn sich beim Datenaustausch zwischen der Erkennungselektronik des medizinischen Geräts und der Einmalverwendungssicherung des Einmalverwendungsartikels ergibt, dass der einmal beschreibbare Register bereits beschieben ist und damit der Einmalverwendungsartikel als bereits benutzt gekennzeichnet ist.

Zusätzlich oder alternativ kann die Einmalverwendungssicherung eine elektrische Leitung aufweisen, die aufgrund eines durch den Empfänger der Einmalverwendungssicherung empfangenen Signals vorzugsweise nicht wiederherstellbar unterbrochen wird, so dass ein Austausch von Daten mit der Erkennungselektronik des medizinischen Geräts unmöglich wird. Dazu sendet die Erkennungselektronik des medizinischen Geräts nach oder während des Betriebs des medizinischen Geräts ein entsprechendes Signal an den Empfänger der Einmalverwendungssicherung am Einmalverwendungsartikel, woraufhin eine elektrische Leitung in der Einmalverwendungssicherung in der Art einer elektrischen Sicherung durchtrennt wird, wodurch eine erneute Verwendung des betreffenden Einmalverwendungsartikels unmöglich wird.

Um die Stromversorgung der am Einmalverwendungsartikel angeordneten Einmalverwendungssicherung ohne Energiespeichermittel zu bewerkstelligen, umfasst die Einmalverwendungssicherung gemäß einer weiteren bevorzugten Ausführungsform induktive Mittel, die eine elektrische Spannung erzeugt, wenn sich die Einmalverwendungssicherung in einem elektromagnetischen Feld befindet. Zweckmäßigerweise wird das elektromagnetische Feld für die Stromversorgung der Einmalverwendungssicherung des Einmalverwendungsartikels durch das medizinische Gerät bzw. die Erkennungselektronik des medizinischen Geräts erzeugt.

Um die Wirksamkeit der Einmalverwendungssicherung zu gewährleisten, ist eine Beseitigung der Einmalverwendungssicherung vom Einmalverwendungsartikel vorzugsweise nur durch Zerstörung des Einmalverwendungsartikels möglich, wodurch der Einmalverwendungsartikel unbrauchbar wird. Die Einmalverwendungssicherung kann beispielsweise derart am Einmalverwendungsartikel angeordnet werden, dass eine Entfernung der Einmalverwendungssicherung bzw. des Transponders vom Verbindungsschlauch nur dann möglich ist, wenn der Verbindungsschlauch aufgeschnitten oder undicht und damit unbrauchbar gemacht wird.

Für einen sicheren Anschluss zwischen dem medizinischen Gerät bzw. der Schlauchpumpe und den sterilen Einmalverwendungsartikeln bzw. den Verbindungsschläuchen, werden die Schläuche über Schlauchverbindungen an der Schlauchpumpe arretiert. Diese Schlauchverbindungen werden in der Regel durch eine Schlauchkupplung hergestellt. Die Anordnung der Einmalverwendungssicherung bzw. des Transponders kann im Verbindungsschlauch selbst oder an einem Kupplungsstück zur Arretierung des Verbindungsschlauches an der Schlauchpumpe erfolgen. Durch die Anordnung des Erkennungssensors der Einmalverwendungssicherung im Kupplungsstück des Verbindungsschlauches, liegt der Erkennungssensor beim Einsatz des Verbindungsschlauchs in unmittelbarer Nähe zum medizinischen Gerät und damit zur Erkennungselektronik des medizinischen Geräts, was einen ungestörten Datenaustausch zwischen dem Erkennungssensor am Verbindungsschlauch und der Erkennungselektronik in der Schlauchpumpe begünstigt.

Um zu verhindern, dass bei einem sterilen Verbindungsschlauch Keime in das Schlauchinnere eindringen, werden die Verbindungsstellen der Schlauchverbindungen in der Regel verklebt. Da der Datenaustausch zwischen dem Erkennungssensor am Verbindungsschlauch und der Erkennungselektronik in der Schlauchpumpe über eine Funkverbindung erfolgt, wird die Kommunikation zwischen dem Erkennungssensor und der Erkennungselektronik durch eine Verklebung der Schlauchverbindungen nicht behindert.

Die oben genannte Aufgabe wird nach der vorliegenden Erfindung ferner durch ein Verfahren zum Betreiben eines oben beschriebenen medizinischen Geräts gelöst, an das mindestens ein Einmalverwendungsartikel der oben beschriebenen Art mit einer elektronischen Einmalverwendungssicherung angeschlossen ist, umfassend die Schritte:
- Austausch von Daten zwischen der elektronischen Einmalverwendungssicherung und einer Erkennungselektronik im medizinischen Gerät,
- Auswertung der zwischen der Einmalverwendungssicherung der Erkennungselektronik ausgetauschten Daten in der Erkennungselektronik des medizinischen Geräts, und
- Steuerung des Betriebs des medizinischen Geräts in Abhängigkeit von der Auswertung der zwischen der Einmalverwendungssicherung der Erkennungselektronik ausgetauschten Daten.

In einer bevorzugten Ausführungsform des erfindungsgemäßen Verfahrens erfolgt vor Beginn des Betriebs des medizinischen Geräts eine Überprüfung, ob ein Datenaustausch zwischen der Einmalverwendungssicherung am Einmalverwendungsartikel und der Erkennungselektronik im medizinischen Gerät überhaupt möglich ist. Wenn das Ergebnis dieser Überprüfung negativ ausfällt, d.h. ein Datenaustausch zwischen der Einmalverwendungssicherung und der Erkennungselektronik im medizinischen Gerät nicht möglich ist, verhindert die Erkennungselektronik den Betrieb des medizinischen Geräts. Auf diese Weise wird gewährleistet, dass bei einer Fehlfunktion im Datenaustausch zwischen der Einmalverwendungssicherung und der Erkennungselektronik der Betrieb des medizinischen Geräts unmöglich ist.

Ein gesundheitliches Risiko kann sich für den Patienten ergeben, wenn die Haltbarkeit der Sterilisation des medizinischen Einmalverwendungsartikels abgelaufen ist oder wenn ein Einmalverwendungsartikel resterilisiert und wiederverwendet wird. Bei einer Ausführungsform der vorliegenden Erfindung erfolgt deshalb eine Überprüfung der aufgrund des Datenaustauschs zwischen der Einmalverwendungssicherung des Einmalverwendungsartikels und der Erkennungselektronik des medizinischen Geräts aus der Einmalverwendungssicherung erhaltenen Daten dahingehend, ob der betreffende Einmalverwendungsartikel bereits verwendet wurde. In Abhängigkeit von dem Ergebnis dieser Überprüfung steuert die Erkennungselektronik den Betrieb des medizinischen Geräts folgendermaßen: wenn die Überprüfung des Dateninhalts des Erkennungssensors durch die Erkennungselektronik ergibt, dass der betreffende Einmalverwendungsartikel noch nicht verwendet wurde, dann lässt die Erkennungselektronik den Betrieb des medizinischen Geräts zu. Wenn die Überprüfung des Dateninhalts des Erkennungssensors durch die Erkennungselektronik ergibt, dass der betreffende Einmalverwendungsartikel bereits verwendet wurde, dann lässt die Erkennungselektronik den Betrieb des medizinischen Geräts nicht zu.

Bei einer weiteren Ausführungsform der vorliegenden Erfindung erfolgt eine Überprüfung der aufgrund des Datenaustauschs zwischen der Einmalverwendungssicherung des Einmalverwendungsartikels und der Erkennungselektronik des medizinischen Geräts aus der Einmalverwendungssicherung erhaltenen Daten dahingehend, ob der Dateninhalt der Einmalverwendungssicherung bereits bei einer vorangegangenen Verwendung des betreffenden Einmalverwendungsartikel verändert wurde, und die Erkennungselektronik bei positivem Ergebnis dieser Überprüfung den Betrieb des medizinischen Geräts verhindert. Das heißt, wenn die Überprüfung des Dateninhalts des Erkennungssensors durch die Erkennungselektronik ergibt, dass der Dateninhalt der Einmalverwendungssicherung bereits bei einer vorangegangenen Verwendung des betreffenden Einmalverwendungsartikel verändert wurde, dann lässt die Erkennungselektronik den Betrieb des medizinischen Geräts nicht zu.

Bei einer weiteren Ausführungsform des Verfahrens nach der vorliegenden Erfindung erfolgt eine Überprüfung der aufgrund des Datenaustauschs zwischen der Einmalverwendungssicherung des Einmalverwendungsartikels und der Erkennungselektronik des medizinischen Geräts aus der Einmalverwendungssicherung erhaltenen Daten dahingehend, ob eine vorgegebene Zeitspanne der Haltbarkeit des betreffenden Einmalverwendungsartikels überschritten ist, wobei die Erkennungselektronik bei positivem Ergebnis dieser Überprüfung den Betrieb des medizinischen Geräts verhindert. Das heißt, wenn die Überprüfung des Dateninhalts des Erkennungssensors durch die Erkennungselektronik ergibt, dass eine vorgegebene Zeitspanne der Haltbarkeit des betreffenden Einmalverwendungsartikels überschritten ist, dann lässt die Erkennungselektronik den Betrieb des medizinischen Geräts nicht zu.

Zusätzlich oder alternativ kann der Betrieb des medizinischen Geräts durch seine Erkennungselektronik aufgrund eines Dateninhalts im Erkennungssensor gesteuert werden, der sich auf die Umgebungsbedingungen während der Lagerung des verwendeten Einmalverwendungsartikels bezieht, wie z.B. die Temperatur oder die Luftfeuchtigkeit, die durch geeignete Sensoren während der Lagerung des betreffenden Einmalverwendungsartikels ermittelt und im Dateninhalt des Erkennungssensors abgespeichert wurden. Dabei wird durch die Erkennungselektronik des medizinischen Geräts aus dem Dateninhalt des Erkennungssensors ermittelt, ob die Umgebungsbedingungen während der Lagerung des betreffenden Einmalverwendungsartikels im zulässigen Bereich waren, wobei die Erkennungselektronik bei negativem Ergebnis dieser Überprüfung den Betrieb des medizinischen Geräts verhindert. Das heißt, wenn die Überprüfung des Dateninhalts des Erkennungssensors durch die Erkennungselektronik ergibt, dass die Umgebungsbedingungen während der Lagerung des betreffenden Einmalverwendungsartikels nicht im zulässigen Bereich waren, dann lässt die Erkennungselektronik den Betrieb des medizinischen Geräts nicht zu.

Zusätzlich oder alternativ kann der Betrieb des medizinischen Geräts durch seine Erkennungselektronik aufgrund eines Dateninhalts im Erkennungssensor gesteuert werden, der sich auf die Art des verwendeten Einmalverwendungsartikels bezieht. Dabei wird durch die Erkennungselektronik des medizinischen Geräts aus dem Dateninhalt des Erkennungssensors ermittelt, ob der betreffende Einmalverwendungsartikel zur Verwendung mit dem betreffenden medizinischen Gerät geeignet ist oder nicht. Bei einer weiteren Ausführungsform des Verfahrens nach der vorliegenden Erfindung erfolgt daher eine Überprüfung der aufgrund des Datenaustauschs zwischen der Einmalverwendungssicherung des Einmalverwendungsartikels und der Erkennungselektronik des medizinischen Geräts aus der Einmalverwendungssicherung erhaltenen Daten dahingehend, ob der betreffende Einmalverwendungsartikel zur Verwendung mit dem medizinischen Gerät geeignet ist, und die Erkennungselektronik bei negativem Ergebnis dieser Überprüfung den Betrieb des medizinischen Geräts verhindert. Das heißt, wenn die Überprüfung des Dateninhalts des Erkennungssensors durch die Erkennungselektronik ergibt, dass der betreffende Einmalverwendungsartikel zur Verwendung mit dem medizinischen Gerät nicht geeignet ist, dann lässt die Erkennungselektronik den Betrieb des medizinischen Geräts nicht zu.

Zusätzlich oder alternativ kann der Betrieb des medizinischen Geräts durch seine Erkennungselektronik aufgrund eines Dateninhalts im Erkennungssensor gesteuert werden, der sich auf einen bestimmten zu behandelnden Patienten bezieht. Dabei wird durch die Erkennungselektronik des medizinischen Geräts aus dem Dateninhalt des Erkennungssensors ermittelt, ob der betreffende Einmalverwendungsartikel zur Verwendung am zu behandelnden Patienten vorgesehen ist oder nicht. Bei einer weiteren Ausführungsform des Verfahrens nach der vorliegenden Erfindung erfolgt daher eine Überprüfung der aufgrund des Datenaustauschs zwischen der Einmalverwendungssicherung des Einmalverwendungsartikels und der Erkennungselektronik des medizinischen Geräts aus der Einmalverwendungssicherung erhaltenen Daten dahingehend, ob der betreffende Einmalverwendungsartikel zur Verwendung am zu behandelnden Patienten vorgesehen ist, und die Erkennungselektronik bei negativem Ergebnis dieser Überprüfung den Betrieb des medizinischen Geräts verhindert. Das heißt, wenn die Überprüfung des Dateninhalts des Erkennungssensors durch die Erkennungselektronik ergibt, dass der betreffende Einmalverwendungsartikel nicht zur Verwendung am zu behandelnden Patienten vorgesehen ist, dann lässt die Erkennungselektronik den Betrieb des medizinischen Geräts nicht zu.

Im Folgenden wird die vorliegende Erfindung anhand von bevorzugten Ausführungsbeispielen unter Bezugnahme auf die beigefügten Zeichnungen näher erläutert. Es zeigen:
- Figur 1: eine teilweise Schnittdarstellung gemäß einer ersten Ausführungsform der vorliegenden Erfindung;
- Figur 2: eine zweite teilweise Schnittdarstellung gemäß der in Figur 1 dargestellten Ausführungsform;
- Figur 3: eine teilweise Schnittdarstellung gemäß einer zweiten Ausführungsform der vorliegenden Erfindung;

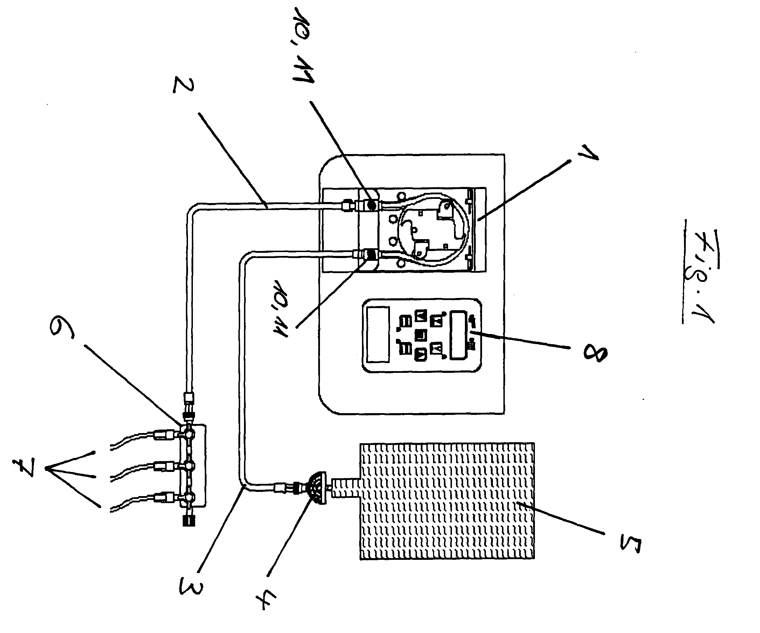

Bei der in Figur 1 gezeigten Ausführungsform der vorliegenden Erfindung ist das medizinische Gerät eine elektrisch betriebene Schlauchpumpe 1, die in Verbindung mit Einmalverwendungsartikeln verwendet wird, um eine Flüssigkeit zu befördern, die in einem Reservoir 5 vorgehalten wird. Bei den Einmalverwendungsartikeln handelt es sich um Verbindungsschläuche 2 und 3, von denen der erste Verbindungsschlauch 3 von dem die zu befördernde Flüssigkeit enthaltenden Reservoir zu der Schlauchpumpe 1 führt, wobei der Verbindungsschlauch 3 mit einem Ende über einen Einstechdorn 4 an das Reservoir 5 angeschlossen ist und an seinem anderen Ende über ein Kupplungsstück 11 mit der Schlauchpumpe 1 verbunden ist. Ein zweiter Verbindungsschlauch 2, führt von der Schlauchpumpe 1 über ein Kupplungsstück 11 zu einer Verteilervorrichtung 6, an die eine Anzahl von Infusionsschläuchen 7 angeschlossen ist. Mit einer solchen Anlage wird dem Patienten beispielsweise bei Infiltrations- oder Tumeszenzverfahren eine Flüssigkeit aus dem Reservoir 5 über die Schlauchpumpe 1 durch die Verbindungsschläuche 2, 3 und über die Infusionsvorrichtung 6, 7 injiziert.

An den Einmalverwendungsartikeln bzw. Verbindungsschläuchen 2 und 3 sind jeweils Einmalverwendungssicherungen 10 in Form eines Erkennungssensors mit elektronischen Mitteln angeordnet. Bei der in den Zeichnungen dargestellten Ausführungsform der vorliegenden Erfindung sind die Einmalverwendungssicherungen bzw. Erkennungssensoren 10 jeweils im Kupplungsstück 11 untergebracht, mit denen die Verbindungsschläuche 2 und 3 mit der Schlauchpumpe 1 verbunden sind. Die Schlauchpumpe 1 ist mit einer Erkennungselektronik 8 ausgestattet, die einen Sender und einen Empfänger für einen Datenaustausch per Funkverbindung aufweist. Die Einmalverwendungssicherungen bzw. Erkennungssensoren 10 umfassen ebenfalls, einen Sender sowie einen Empfänger zum Austausch von Daten zwischen der Einmalverwendungssicherung 10 und der Erkennungselektronik 8 im medizinischen Gerät 1 über eine drahtlose Funkverbindung.

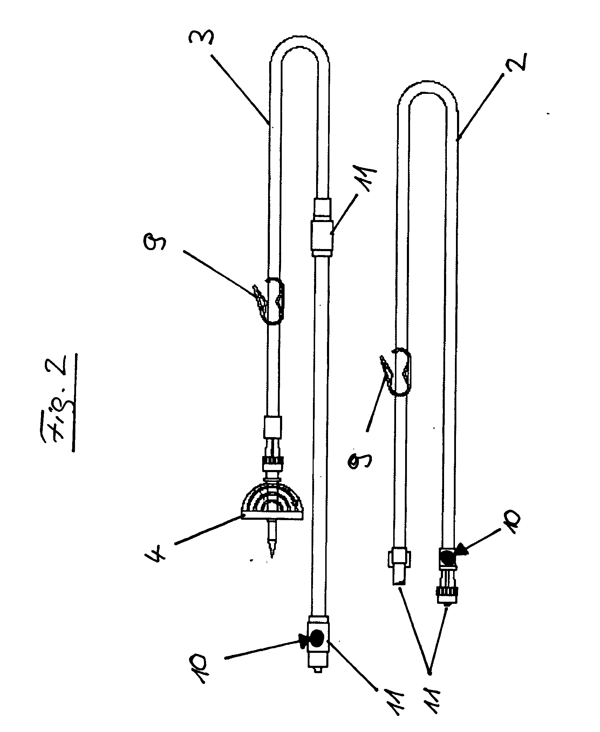

Figur 2 zeigt eine Detailansicht der Einmalverwendungsartikel bzw. Verbindungsschläuche 2 und 3 von Figur 1. Beide Verbindungsschläuche 2, 3 sind jeweils mit einer Schlauchklemme 9 ausgestattet, die im geschlossenen Zustand einen Durchfluss von Flüssigkeit durch den betreffenden Verbindungsschlauch unterbinden. In Figur 2 sind die Schlauchklemmen 9 im geöffneten Zustand dargestellt, so dass ein Durchfluss von Flüssigkeit durch den betreffenden Verbindungsschlauch 2, 3 möglich ist. Der erste Verbindungsschlauch 3 ist an einem Ende mit dem Einstechdorn 4 zum Anschluss an das Reservoir 5 ausgestattet und an seinem anderen Ende mit einem Kupplungsstück 11, über das der Verbindungsschlauch 3 mit der Schlauchpumpe 1 verbunden wird. Der zweite Verbindungsschlauch 2, ist an einem Ende mit einem Kupplungsstück 11 zum Anschluss des Verbindungsschlauchs 2 an eine Infusionsvorrichtung 6, 7 ausgestattet und an seinem anderen Ende mit einem Kupplungsstück 11, über das der Verbindungsschlauch 2 mit der Schlauchpumpe 1 verbunden wird.

In den Kupplungsstücken 11, über die der Verbindungsschlauch 2, 3 an das medizinische Gerät 1 angeschlossen wird , ist jeweils die eine Einmalverwendungssicherung in Form eines elektronischen Erkennungssensors 10, wie z.B. ein Transponder, unverlierbar angeordnet. Zwischen der Erkennungselektronik 8 der Schlauchpumpe 1 und dem Erkennungssensor 10 kann über die jeweiligen Sender und Empfänger ein Datenaustausch mittels drahtloser Funkverbindung stattfinden. Der Erkennungssensor 10 ist in dem Kupplungsstück 11 des Verbindungsschlauchs 2, 3 so integriert, dass eine Beseitigung des Erkennungssensors 10 vom Verbindungsschlauch 2, 3 nur durch Zerstörung des betreffenden Verbindungsschlauchs möglich ist, wodurch der Einmalverwendungsartikel 2, 3 unbrauchbar wird.

Der Erkennungssensor 10 umfasst elektronische Speichermittel, deren Dateninhalt durch die Erkennungselektronik 8 der Schlauchpumpe 1 nach oder während des Betriebs des medizinischen Geräts 1 verändert werden kann. Auf diese Weise kann die Erkennungselektronik 8 nach oder während des Betriebs des medizinischen Geräts 1 den Dateninhalt der elektronischen Speichermittel im Erkennungssensor 10 dahingehend verändern, dass der Einmalverwendungsartikel benutzt wurde und dadurch den Einmalverwendungsartikel als unbrauchbar für einen weiteren Gebrauch kennzeichnen.

Die Erkennungselektronik 8 der Schlauchpumpe 1 führt vor Beginn des eigentlichen Infusionsbetriebs eine Überprüfung der in den elektronischen Speichermitteln des im Erkennungssensor 10 gespeicherten Daten durch, um zu ermitteln, ob der betreffende Verbindungsschlauch 2, 3 bereits bei einer vorangegangenen Verwendung benutzt wurde. Wenn die Überprüfung des Dateninhalts des Erkennungssensors 10 zu dem Ergebnis führt, dass der betreffende Verbindungsschlauch 2, 3 bereits benutzt wurde, so verhindert die Erkennungselektronik 8 den Pumpbetrieb der Schlauchpumpe 1. Auf diese Weise kann mit Hilfe der erfindungsgemäßen Einmalverwendungsartikel 2, 3 mit Einmalverwendungssicherung 10 sichergestellt werden, dass der Einmalverwendungsartikel 2, 3 tatsächlich nur einmal verwendet wird, was eine erhöhte Sicherheit für den Patienten mit sich bringt.

Der Erkennungssensor 10 umfasst ferner elektronische Speichermittel mit mindestens einem vorzugsweise wiederbeschreibbaren elektronischen Register, in dem Daten gespeichert und verändert werden können. In solchen elektronischen Registern können beispielsweise Informationen gespeichert, die sich auf eine vorangegangene Benutzung, auf eine Zeitspanne der Haltbarkeit, auf die Umgebungsbedingungen während der Lagerung, auf die Eignung des betreffenden Einmalverwendungsartikels für das medizinische Gerät und/oder auf den zu behandelnden Patienten beziehen. Ferner kann der Erkennungssensor der Einmalverwendungssicherung zumindest einen elektronischen Register umfassen, der nur einmal beschreibbar ist. Dadurch kann die Erkennungselektronik den Betrieb des medizinischen Geräts verhindern, wenn sich beim Datenaustausch zwischen der Erkennungselektronik des medizinischen Geräts und der Einmalverwendungssicherung des Einmalverwendungsartikels ergibt, dass der einmal beschreibbare Register bereits beschieben ist und damit der Einmalverwendungsartikel als bereits benutzt gekennzeichnet ist.

Die Erkennungselektronik 8 der Schlauchpumpe 1 erzeugt ein elektromagnetisches Feld, das bis zum Erkennungssensor 10 der an die Schlauchpumpe 1 angeschlossenen Verbindungsschläuche 2, 3 reicht. Die Einmalverwendungssicherung 10 umfasst induktive Mittel, wie z.B. eine Spule, die eine elektrische Spannung erzeugt, wenn sich die Einmalverwendungssicherung in dem von der Erkennungselektronik 8 der Schlauchpumpe erzeugten elektromagnetischen Feld befindet. Aufgrund der durch die induktiven Mittel erzeugten chen 2, 3 angeordneten Erkennungssensoren 10 ohne Energiespeichermittel mit Strom versorgt werden, um den Datenaustausch mit der Erkennungselektronik 8 und die Veränderung des Dateninhalts des Erkennungssensors 10 zu ermöglichen.

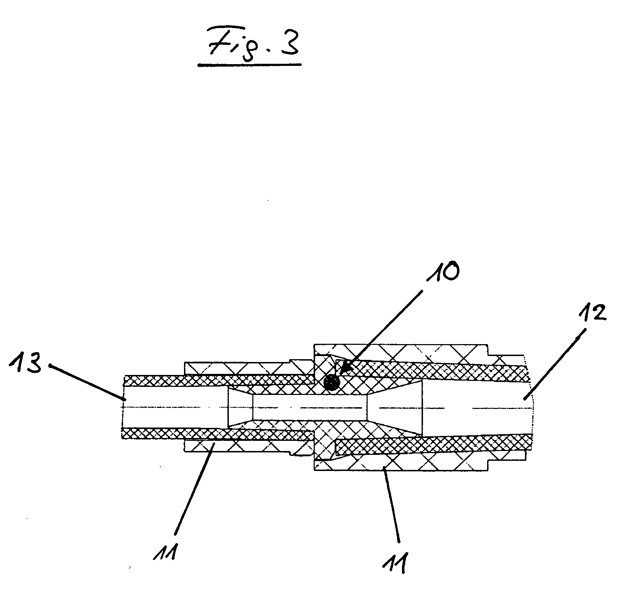

Figur 3 ist eine Detailansicht von Einmalverwendungsartikeln, die eine Kupplung zwischen einem Verbindungsschlauch 13 und einen daran angeschlossenen Verlängerungsschlauch 12 zeigt. Die Kupplung setzt sich auf der einen Seite aus dem Kupplungsstück 11 am Ende des Verbindungsschlauchs 12 und auf der anderen Seite aus dem Kupplungsstück 11 am Ende eines Verlängerungsschlauchs 13 zusammen. Im Kupplungsstück 11 am Ende des Verlängerungsschlauchs 13 ist ein Erkennungssensor 10 integriert, der die oben beschriebenen Merkmale und Eigenschaften aufweist. Außerdem ist im Kupplungsstück am Ende des Verbindungsschlauchs 12 ein Erkennungssensor 10 integriert (im Figur 3 nicht dargestellt), der ebenfalls die oben beschriebenen Merkmale und Eigenschaften aufweist. Neben dem Datenaustausch mit dem Erkennungssensor, der am Verbindungsschlauch 12 angeordnet ist, führt die Erkennungselektronik 8 der Schlauchpumpe 1 auch einen Datenaustausch mit dem Erkennungssensor 10, der am Verlängerungsschlauch 13 angeordnet ist, durch. Falls zwar der Verbindungsschlauch 12 den Anforderungen für eine erste Benutzung entspricht, der Verlängerungsschlauch 13 den Anforderungen für eine erste Benutzung jedoch nicht entspricht, weil er beispielsweise bereits benutzt wurde, verhindert die Erkennungselektronik 8 den Betrieb der Schlauchpumpe 1. Auf diese Weise kann verhindert werden, dass die Schlauchpumpe 1 mit einem Verbindungsschlauch 12 betrieben wird, der durch einen benutzten Verlängerungsschlauch 13 verlängert wurde. Ebenso ist mit Hilfe der vorliegenden Erfindung unmöglich, die Schlauchpumpe 1 mit einem benutzten Verbindungsschlauch 12 zu betreiben, der durch einen unbenutzten Verlängerungsschlauch 13 verlängert wurde.

### Liste der Bezugszeichen

- 1: Medizinisches Gerät bzw. Schlauchpumpe
- 2: Einmalverwendungsartikel bzw. Verbindungsschlauch
- 3: Einmalverwendungsartikel bzw. Verbindungsschlauch
- 4: Einstechdorn
- 5: Reservoir
- 6: Verteiler
- 7: Infusionsschläuche
- 8: Erkennungselektronik
- 9: Schlauchklemmen
- 10: Einmalverwendungssicherung bzw. Erkennungssensor (Transponder)
- 11: Kupplungsstück
- 12: Verlängerungsschlauch
- 13: Verbindungsschlauch

## Patentansprüche

1. Medizinisches Gerät, das in Verbindung mit mindestens einem Einmalverwendungsartikel (2, 3) elektrisch betreibbar ist, **dadurch gekennzeichnet, dass** das medizinische Gerät eine Erkennungselektronik (8) mit einem Sender und einem Empfänger aufweist und der mindestens eine Einmalverwendungsartikel (2, 3) eine Einmalverwendungssicherung (10) mit elektronischen Mitteln aufweist, die einen Sender und einen Empfänger zum Austausch von Daten zwischen der Einmalverwendungssicherung (10) und der Erkennungselektronik (8) im medizinischen Gerät (1) umfassen, wobei die Erkennungselektronik (8) in Abhängigkeit von einer Auswertung der zwischen der Einmalverwendungssicherung (10) der Erkennungselektronik (8) ausgetauschten Daten den Betrieb des medizinischen Geräts (1) steuert.

2. Medizinisches Gerät nach Anspruch 1, wobei das medizinische Gerät (1) eine elektrisch betriebene Schlauchpumpe zur Beförderung von Fluiden ist und der mindestens eine Einmalverwendungsartikel (2, 3) einen Verbindungsschlauch (3) umfasst, der von einem das zu befördernde Fluid enthaltenden Reservoir (5) zu der Schlauchpumpe (1) führt, und/oder einen Verbindungsschlauch (2) umfasst, der von der Schlauchpumpe (1) zu einer Infusionsvorrichtung führt.

3. Medizinisches Gerät nach einem der Ansprüche 1 oder 2, wobei die Erkennungselektronik (8) des medizinischen Geräts (1) mit einer elektronischen Steuerung des medizinischen Geräts (1) verbunden ist.

4. Einmalverwendungsartikel, der in Verbindung mit einem medizinischen Gerät (1) nach einem der vorangehenden Ansprüche einsetzbar ist, **dadurch gekennzeichnet, dass** der Einmalverwendungsartikel (2, 3) eine Einmalverwendungssicherung (10) mit elektronischen Mitteln aufweist, die einen Sender und einen Empfänger zum Austausch von Daten zwischen der Einmalverwendungssicherung (10) und einer Erkennungselektronik (8) im medizinischen Gerät (1) umfassen.

5. Einmalverwendungsartikel nach Anspruch 4, wobei die Einmalverwendungssicherung (10) des Einmalverwendungsartikels (2, 3) einen elektronische Erkennungssensor, beispielsweise einen Transponder umfasst, der mit dem Einmalverwendungsartikel (2, 3) vorzugsweise unverlierbar verbunden ist.

6. Einmalverwendungsartikel nach einem der Ansprüche 4 oder 5, wobei die Einmalverwendungssicherung (10) elektronische Speichermittel mit mindestens einem wiederbeschreibbaren elektronischen Register zum Speichern von Daten umfasst.

7. Einmalverwendungsartikel nach einem der vorangehenden Ansprüche, wobei die Einmalverwendungssicherung (10) des Einmalverwendungsartikels (2, 3) elektronische Speichermittel umfasst, deren Dateninhalt durch die Erkennungselektronik (8) während des Betriebs des medizinischen Geräts (1) verändert wird.

8. Einmalverwendungsartikel nach einem der vorangehenden Ansprüche, wobei die Einmalverwendungssicherung (10) des Einmalverwendungsartikels (2, 3) elektronische Speichermittel umfasst zum Speichern von Informationen bezüglich einer vorangegangenen Benutzung des betreffenden Einmalverwendungsartikels (2, 3) und/oder bezüglich einer Zeitspanne der Haltbarkeit des betreffenden Einmalverwendungsartikels und/oder bezüglich der Umgebungsbedingungen während der Lagerung des betreffenden Einmalverwendungsartikels und/oder bezüglich der Eignung des betreffenden Einmalverwendungsartikels für das medizinische Gerät (1) und/oder bezüglich des zu behandelnden Patienten.

9. Einmalverwendungsartikel nach Anspruch 8, wobei der elektronische Speicher der Einmalverwendungssicherung (10) zumindest einen Register umfasst, der nur einmal beschreibbar ist.

10. Einmalverwendungsartikel nach einem der vorangehenden Ansprüche, wobei die Einmalverwendungssicherung (10) eine elektrische Leitung umfasst, die aufgrund eines durch den Empfänger der Einmalverwendungssicherung (10) empfangenen Signals vorzugsweise nicht wiederherstellbar unterbrochen wird, so dass ein Austausch von Daten mit der Erkennungselektronik (8) des medizinischen Geräts (1) unmöglich wird.

11. Einmalverwendungsartikel nach einem der vorangehenden Ansprüche, wobei die Einmalverwendungssicherung (10) induktive Mittel umfasst, die eine elektrische Spannung zur Stromversorgung der Einmalverwendungssicherung (10) erzeugt, wenn sich die Einmalverwendungssicherung (10) in einem elektromagnetischen Feld befindet.

12. Einmalverwendungsartikel nach einem der vorangehenden Ansprüche, wobei das medizinische Gerät (1) bzw. die Erkennungselektronik (8) des medizinischen Geräts (1) ein elektromagnetischen Feld für die Stromversorgung der Einmalverwendungssicherung (10) des Einmalverwendungsartikels (2, 3) erzeugt.

13. Einmalverwendungsartikel nach einem der vorangehenden Ansprüche, wobei der Austausch von Daten zwischen der Einmalverwendungssicherung (10) und der Erkennungselektronik (8) drahtlos über eine Funkverbindung mittels elektromagnetischer Wellen erfolgt.

14. Einmalverwendungsartikel nach einem der vorangehenden Ansprüche, wobei die Einmalverwendungssicherung (10) im Einmalverwendungsartikel (2, 3) derart angeordnet ist, dass durch eine Beseitigung der Einmalverwendungssicherung (10) vom Einmalverwendungsartikel (2, 3) der Einmalverwendungsartikel (2, 3) unbrauchbar wird.

15. Einmalverwendungsartikel nach einem der vorangehenden Ansprüche, wobei die Einmalverwendungssicherung (10) im Verbindungsschlauch selbst oder an einem Kupplungsstück zur Arretierung des Verbindungsschlauchs an der Schlauchpumpe angeordnet ist.

16. Verfahren zum Beteiben eines medizinischen Geräts (1) nach einem der vorangehenden Ansprüche, an das mindestens ein Einmalverwendungsartikel (2, 3) mit einer elektronischen Einmalverwendungssicherung (10) angeschlossen ist, umfassend die Schritte:
• Austausch von Daten zwischen der elektronischen Einmalverwendungssicherung (10) und einer Erkennungselektronik (8) im medizinischen Gerät (1),
• Auswertung der zwischen der Einmalverwendungssicherung (10) der Erkennungselektronik (8) ausgetauschten Daten in der Erkennungselektronik (8) des medizinischen Geräts (1), und
• Steuerung des Betriebs des medizinischen Geräts (1) in Abhängigkeit von der Auswertung der zwischen der Einmalverwendungssicherung (10) der Erkennungselektronik (8) ausgetauschten Daten.

17. Verfahren nach Anspruch 16, wobei vor Beginn des Betriebs des medizinischen Geräts (1) eine Überprüfung erfolgt, ob ein Datenaustausch zwischen der Einmalverwendungssicherung (10) und der Erkennungselektronik (8) im medizinischen Gerät (1) möglich ist, und die Erkennungselektronik (8) bei negativem Ergebnis dieser Überprüfung den Betrieb des medizinischen Geräts (1) verhindert.

18. Verfahren nach einem der Ansprüche 16 oder 17, wobei eine Überprüfung der aufgrund des Datenaustauschs zwischen der Einmalverwendungssicherung (10) des Einmalverwendungsartikels (2, 3) und der Erkennungselektronik (8) des medizinischen Geräts (1) aus der Einmalverwendungssicherung (10) erhaltenen Daten dahingehend erfolgt, ob der betreffende Einmalverwendungsartikel (2, 3) bereits verwendet wurde, und die Erkennungselektronik (8) bei positivem Ergebnis dieser Überprüfung den Betrieb des medizinischen Geräts (1) verhindert.

19. Verfahren nach einem der vorangehenden Ansprüche 16 bis 18, wobei eine Überprüfung der aufgrund des Datenaustauschs zwischen der Einmalverwendungssicherung (10) des Einmalverwendungsartikels (2, 3)s und der Erkennungselektronik (8) des medizinischen Geräts (1) aus der Einmalverwendungssicherung (10) erhaltenen Daten dahingehend erfolgt, ob der Dateninhalt der Einmalverwendungssicherung (10) bereits bei einer vorangegangenen Verwendung des betreffenden Einmalverwendungsartikel (2, 3) verändert wurde, und die Erkennungselektronik (8) bei positivem Ergebnis dieser Überprüfung den Betrieb des medizinischen Geräts (1) verhindert.

20. Verfahren nach einem der vorangehenden Ansprüche 16 bis 19, wobei eine Überprüfung der aufgrund des Datenaustauschs zwischen der Einmalverwendungssicherung (10) des Einmalverwendungsartikels (2, 3) und der Erkennungselektronik (8) des medizinischen Geräts (1) aus der Einmalverwendungssicherung (10) erhaltenen Daten dahingehend erfolgt, ob eine vorgegebene Zeitspanne der Haltbarkeit des betreffenden Einmalverwendungsartikels (2, 3) überschritten ist, und die Erkennungselektronik (8) bei positivem Ergebnis dieser Überprüfung den Betrieb des medizinischen Geräts (1) verhindert.

21. Verfahren nach einem der vorangehenden Ansprüche 16 bis 20, wobei eine Überprüfung der aufgrund des Datenaustauschs zwischen der Einmalverwendungssicherung (10) des Einmalverwendungsartikels (2, 3) und der Erkennungselektronik (8) des medizinischen Geräts (1) aus der Einmalverwendungssicherung (10) erhaltenen Daten dahingehend erfolgt, ob vorgegebene Umgebungsbedingungen während der Lagerung des betreffenden Einmalverwendungsartikels (2, 3) eingehalten wurden, wie z.B. die Temperatur oder die Luftfeuchtigkeit, die durch geeignete Sensoren während der Lagerung des betreffenden Einmalverwendungsartikels (2, 3) ermittelt und im Dateninhalt des Erkennungssensors abgespeichert wurden, und die Erkennungselektronik (8) bei negativem Ergebnis dieser Überprüfung den Betrieb des medizinischen Geräts (1) verhindert.

22. Verfahren nach einem der vorangehenden Ansprüche 16 bis 21, wobei eine Überprüfung der aufgrund des Datenaustauschs zwischen der Einmalverwendungssicherung (10) des Einmalverwendungsartikels (2, 3) und der Erkennungselektronik (8) des medizinischen Geräts (1) aus der Einmalverwendungssicherung (10) erhaltenen Daten dahingehend erfolgt, ob der betreffende Einmalverwendungsartikel (2, 3) zur Verwendung mit dem medizinischen Gerät (1) geeignet ist, und die Erkennungselektronik (8) bei negativem Ergebnis dieser Überprüfung den Betrieb des medizinischen Geräts (1) verhindert.

23. Verfahren nach einem der vorangehenden Ansprüche 16 bis 22, wobei eine Überprüfung der aufgrund des Datenaustauschs zwischen der Einmalverwendungssicherung (10) des Einmalverwendungsartikels (2, 3) und der Erkennungselektronik (8) des medizinischen Geräts (1) aus der Einmalverwendungssicherung (10) erhaltenen Daten dahingehend erfolgt, ob der betreffende Einmalverwendungsartikel (2, 3) zur Verwendung am zu behandelnden Patienten vorgesehen ist, und die Erkennungselektronik (8) bei negativem Ergebnis dieser Überprüfung den Betrieb des medizinischen Geräts (1) verhindert.

24. Verfahren nach einem der vorangehenden Ansprüche 16 bis 23, wobei der Dateninhalt der Einmalverwendungssicherung (10) des Einmalverwendungsartikels (2, 3) während des Betriebs des medizinischen Geräts (1) durch die Erkennungselektronik (8) des medizinischen Geräts (1) verändert wird.

25. Verfahren nach einem der vorangehenden Ansprüche 16 bis 24, wobei die Erkennungselektronik (8) des medizinischen Geräts (1) den Dateninhalt der Einmalverwendungssicherung (10) des Einmalverwendungsartikels (2, 3) nach dem Betrieb des medizinischen Geräts (1) mit dem betreffenden Einmalverwendungsartikel (2, 3) eine elektrische Leitung in der Einmalverwendungssicherung (10) vorzugsweise nicht wiederherstellbar unterbricht, so dass ein Austausch von Daten mit der Erkennungselektronik (8) des medizinischen Geräts (1) unmöglich wird.
